# EUROPÄISCHE PATENTANMELDUNG

(11) **EP 2 072 046 A1**
(43) Veröffentlichungstag der Anmeldung: **24.06.2009**
(21) Anmeldenummer: 07024993.3
(22) Anmeldetag: 21.12.2007
(51) Int. Cl.: A61K 31/191, A61K 33/10, A61P 35/00, A61P 9/12, A61P 19/10, A61P 13/12

(54) **Verwendung einer hydrocarbonathaltigen Zubereitung zur Prävention oder Behandlung von Herz-Kreislauf-Erkrankungen, Diabetes, Osteoporose und chronischem Nierenversagen**

(71) Anmelder: Kopp, K.F., Prof. Dr., 85560 Ebersberg (DE)
(72) Erfinder: Kopp, K.F., Prof. Dr., 85560 Ebersberg (DE)
(74) Vertreter: Weiss, Wolfgang

(57) **Zusammenfassung**

Die Erfindung betrifft die Verwendung einer Zubereitung, enthaltend ein Hydrogencarbonatsalz oder eine Verbindung, die im Körper zu einem Hydrogencarbonatsalz metabolisiert wird, zur Prävention oder Behandlung von Herz-Kreislauf-Erkrankungen, Bluthochdruck, Diabetes, Osteoporose und zur Abwendung eines im Rahmen anderer Erkrankungen möglichen akuten Nierenversagens sowie von chronischem Nierenversagen, wobei die Zubereitung zur Verabreichung in einer Dosierung vorgesehen ist, durch die der pH des Urins auf einen Wert ≥ pH 7,0 eingestellt wird.

## Beschreibung

Die Erfindung betrifft die Verwendung einer Zubereitung, enthaltend ein Hydrogencarbonatsalz oder eine Verbindung, die im Körper zu einem Hydrogencarbonatsalz metabolisiert wird, zur Prävention oder Behandlung von Herz-Kreislauf-Erkrankungen, Krebserkrankungen, Bluthochdruck, Diabetes, Osteoporose, chronischem Nierenversagen und zur Abwendung eines im Rahmen anderer Erkrankungen möglichen akuten Nierenversagens, wobei die Zubereitung zur Verabreichung in einer Dosierung vorgesehen ist, durch die der pH des Urins auf einen Wert ≥ pH 7,0 eingestellt wird.

Herz-Kreislauf-Erkrankungen, Krebserkrankungen, Bluthochdruck, Diabetes, Osteoporose und chronisches Nierenversagen sind Zivilisations- bzw. Volkskrankheiten, von denen eine große Zahl der Bevölkerung betroffen ist.

Häufig hängt das Entstehen dieser Krankheiten auch mit einer gesundheitsschädlichen Lebensweise, wie z.B. Überernährung und Bewegungsmangel, die zur Störung des Säure-Basen-Haushalts führen, zusammen. Der volkswirtschaftliche Schaden, der durch diese Volkskrankheiten entsteht, ist beträchtlich.

Normalerweise wird das Säure-Basen-Gleichgewicht im Organismus dadurch konstant gehalten, dass die Nieren assistiert von den Lungen, die täglich im Stoffwechsel entstehenden Säuren (ca. 50 mmol/Tag) vermittels des sauren Urins ausscheiden. Gleichzeitig wird die Konzentration von Natriumhydrogencarbonat im Blut durch Rückresorption von Natriumhydrogencarbonat aus dem Primärharn und durch Neusynthese auf dem physiologischen Niveau von 25 mmol/l konstant gehalten. Daher treten vor allem bei Patienten mit Nierenerkrankungen Probleme mit dem Säure-Basen-Haushalt auf, d.h. es kommt zur Übersäuerung (= Azidose). Die erkrankte Niere kann weder die anfallenden Stoffwechselsäuren im notwendigen Maße ausscheiden, noch kann sie dem Organismus ausreichend Natriumhydrogencarbonat zurückgewinnen.

Der natürliche, physiologische Alterungsprozess verursacht jedoch auch beim Gesunden früher oder später eine Azidose. In der Medizin kann der altersbedingte Leistungsverlust der Nieren mit der so genannten Cockcroft-Formel genau berechnet werden. So nimmt nach dem 25. Lebensjahr die Leistung der Nieren kontinuierlich ab und beträgt zwischen dem 60. und dem 70. Lebensjahr nur noch etwa 50 %. Zwar wäre durch eine überwiegend vegetarische Nahrung eine Alkalisierung des Organismus u.a. durch Citrate, die im Körper durch Natriumhydrogencarbonat (ca. 60 mmol/Tag) verstoffwechselt werden, möglich. Ein Nachteil dieser vegetarischen Ernährung zur Prävention und Behandlung der Azidose sind jedoch Eiweiß- und/oder andere Mangelerscheinungen, die beispielsweise bei Reisbauern in China und Indien auftreten.

Ursächlich und entscheidend für das Bestehen einer chronischen Azidose in einem überwiegenden Teil der modernen Zivilisations-Bevölkerung ist, dass die o.g. modernen Lebensgewohnheiten, und davon stellt die Ernährung einen Teilfaktor dar, die Leistungsfähigkeit der Nieren als Ausscheidungs- und als Regulationsorgan des Säure-Basen-Haushaltes überfordern. Die bestehende evolutionsbedingte anatomische Struktur und Funktion der Nieren befähigt diese allenfalls die Stoffwechselanforderungen zu erfüllen, wie sie durch die Umwelt- und Lebensbedingungen des Steinzeitmenschen gegeben waren. Plausibel wird dies auch bei der Betrachtung der etwas bekannteren anatomischen und funktionellen Gegebenheiten unseres Zahnapparates und des gesamten Verdauungstraktes, die ebenfalls noch so wie für die steinzeitlichen Lebensbedingungen angelegt sind. Gegenüber den heutigen geänderten Anforderungen der Zivilisations-Umwelt besteht eine latente Insuffizienz der Nieren speziell bezüglich des Säure-Basen-Haushaltes. Saure Stoffwechsel-Endprodukte werden einerseits in erhöhtem Umfang im Organismus produziert und andererseits unzureichend renal ausgeschieden. Somit besteht permanent ein Säureüberschuss, d.h. eine Azidose im Organismus. Durch die physiologische Alterung und der damit noch verstärkten Leistungsminderung der Nieren, speziell in Altersbereichen, die der Steinzeitmensch nie erreicht hat, werden die daraus entstehenden Folgeschäden besonders deutlich. Die chronisch latente und altersbedingt zunehmende Übersäuerung bildet neben anderen, genetischen Faktoren daher eine Vorbedingung für die genannten Volks- oder Zivilisationskrankheiten.

Daher ist eine kostengünstige Prävention und Behandlung von Zivilisationskrankheiten, z.B. Krankheiten, wie Herz-Kreislauf-Erkrankungen, Krebserkrankungen, Bluthochdruck, Diabetes, Osteoporose und chronischem Nierenversagen, die auch durch eine Azidose bedingt sein können, von großer Bedeutung.

Aus EP 0 439 061 A1 sind wässrige Lösungen bekannt, die zur Herstellung einer Lösung zur intravenösen Medikation zur Behandlung von Patienten, die unter Nieren-Dysfunktion oder Nierenversagen leiden, verwendet werden und um das Säure/Basen-Gleichgewicht zu stabilisieren. Diese intravenösen Lösungen werden allgemein bei der Behandlung von Patienten vor, während und nach einem chirurgischen Eingriff oder bei Störungen der Gesundheit und des Stoffwechsels, wie z.B. bei Chemotherapien, welche auch zu Störungen der Nierenfunktion führen können, angewendet.

Ein Nachteil dieser Lösungen ist, dass diese nur in der Klinik verabreicht werden können und dass diese daher nicht zur täglichen Prävention oder einfachen Behandlung der Patienten geeignet sind.

Aufgabe der vorliegenden Erfindung war es, die Nachteile des Standes der Technik zumindest teilweise zu überwinden.

Diese Aufgabe wird gelöst durch die Verwendung einer Zubereitung, enthaltend als Wirkstoff mindestens ein Hydrogencarbonatsalz oder/und mindestens eine Verbindung, die im Körper zu einem Hydrogencarbonatsalz metabolisiert wird, zur Herstellung eines Mittels zur Prävention oder Behandlung von Zivilisationskrankheiten wie etwa Herz-Kreislauf-Erkrankungen, Krebserkrankungen, insbesondere zur Verringerung oder/und Verhinderung der Metastasenbildung, z.B. nach einem chirurgischen Eingriff oder/und im Rahmen einer Strahlen- oder Chemotherapie, Bluthochdruck, Diabetes, Osteoporose, chronischem Nierenversagen und zur Abwendung eines im Rahmen anderer Erkrankungen möglichen akuten Nierenversagens, wobei die Zubereitung zur Verabreichung in einer Dosierung vorgesehen ist, durch die der pH des Urins auf einen Wert ≥ pH 7,0, vorzugsweise pH 7,0 bis 7,5, eingestellt wird.

Dabei wird die oben genannte Zubereitung vorzugsweise in einem Verfahren verwendet, das die folgenden Schritte umfasst:
(a) Verabreichung der oben definierten Zubereitung an eine Person, z:B. an einen Patienten,
(b) Messung des pH-Wertes des Urins der Person und
(c) erneute Verabreichung einer oben genannten Zubereitung, falls der pH des Urins < pH 7,0 beträgt, und Wiederholung der Verabreichung und Messung des pH des Urins, bis der pH des Urins ≥ pH 7,0 beträgt.

Vorzugsweise ist die Zusammensetzung für eine langfristige Verabreichung von 1 Monat oder länger, z.B. 6 Monate, 12 Monate oder länger, vorgesehen. Weiterhin ist bevorzugt, dass der pH des Urins durch eine kontinuierliche, z.B. tägliche, Verabreichung permanent auf einen Wert ≥ pH 7,0 eingestellt wird.

In einer bevorzugten Ausführungsform wird die Zubereitung daher für eine ausreichende Zeitdauer verabreicht, um eine permanente Einstellung des Urin-pH-Wertes zu erreichen, d.h. die Verfahrensschritte (a) bis (c) werden so oft wiederholt, bis der pH des Urins in einem Bereich von pH 7,0 bis pH 7,5 liegt. Dieser pH-Bereich von pH 7,0 bis pH 7,5 wird als Kopp'scher pH-Bereich definiert.

Ein großer Vorteil des erfindungsgemäßen Verfahrens ist, dass dieses einfach von jedermann durch die regelmäßige, z.B. tägliche Einnahme des Mittels und gegebenenfalls mehrfache, z.B. tägliche, Kontrolle des pH-Wertes des Urins durchgeführt werden kann. Dabei war es überraschend, dass bei erfindungsgemäßer permanenter Einstellung des pH-Wertes auf ≥ pH 7,0 eine dauerhafte positive Wirkung erzielt wird, da bisher allgemein angenommen wurde, dass eine permanente Alkalisierung des pH-Wertes des Urins schädlich ist (z.B. Kraske, "Säure-Basen Balance", GU-Verlag).

Für die Zubereitungen kommen Wirkstoffe in Betracht, die ein Hydrogencarbonatsalz oder/und eine Verbindung enthalten, die im Körper zu einem Hydrogencarbonatsalz metabolisiert wird, z.B. ein Citratsalz. Bevorzugt sind Zubereitungen enthaltend Natriumhydrogencarbonat, Kaliumhydrogencarbonat, Calciumhydrogencarbonat, Natriumcitrat, Kaliumcitrat, Calciumcitrat, Magnesiumcitrat oder Kombinationen davon. Besonders bevorzugt sind Zubereitungen, die als Wirkstoff (i) Natriumhydrogencarbonat oder/und Natriumcitrat, (ii) Kaliumcitrat, (iii) Calciumcitrat und (iv) Magnesiumcitrat enthalten.

Als Zubereitungsformen kommen orale Präparate in Betracht, z.B. Präparate, welche den oder die Wirkstoffe alleine oder Präparate, die außerdem noch Zusatzstoffe enthalten, z.B. Nahrungsmittel oder Nahrungsergänzungsmittel, wie Getränke, Pulver, Bonbons, Tabletten, Dragees, Kapseln oder Riegel, die neben den Wirkstoffen Nährstoffe oder/und Vitamine enthalten. Als Getränke kommen alle herkömmlichen Getränke, insbesondere Tafelwasser, Limonaden, Fruchtsäfte oder Energie-Drinks in Betracht. Als Riegel kommen Kraftriegel oder Müsliriegel in Betracht. Bevorzugt ist die Verwendung von Tabletten oder Dragees. Die Tabletten oder Dragees können dabei einen Überzug zur besseren Schluckbarkeit aufweisen, z.B. einen Überzug aus Eudragit. Der Überzug kann gegebenenfalls farbig oder/und mit Geschmacksstoffen versehen sein.

Vorzugsweise enthalten die Zubereitungen kationische Elektrolyte, insbesondere Na, K, Ca und Mg, im Wesentlichen entsprechend dem Verhältnis ihres Vorkommens im Humanserum (Kopp'sche Tablette). Bevorzugte kationische Elektrolyte sind Natrium, Kalium, Calcium, Magnesium, als Hydrogencarbonate oder als Citrate. Das Molverhältnis Mg:Ca:K:Na beträgt vorzugsweise 1:1,5-5:3-10:100-250. Natrium liegt vorzugsweise als natriumhydrogencarbonat oder/und -citrat vor. Kalium, Calcium und Magnesium liegen vorzugsweise als Citrate vor.

Eine typische Dosis der Zubereitung enthält 0,5-1,5 g Natriumhydrogencarbonat, bevorzugt 0,8-1,3 g Natriumhydrogencarbonat, besonders bevorzugt 0,9-1,1 g Natriumhydrogencarbonat (oder entsprechend andere Wirkstoffe). Diese Dosis kann - nach Bedarf - z.B. täglich einmal oder mehrmals verabreicht werden.

Neben den genannten Wirkstoffen kann die Zubereitung darüber hinaus auch Spurenelemente wie Eisen, Mangan, Selen, Zink oder/und Kupfer enthalten.

Die erfindungsgemäße Zubereitung ist geeignet, den pH des Urins auf einen Wert ≥ pH 7,0 einzustellen, bevorzugt um einen pH des Urins einzustellen, der im Bereich von pH 7,0 bis pH 7,5, d.h. im Kopp'schen pH-Bereich liegt.

Die tägliche Messung des pH-Wertes des Urins, die gegebenenfalls mehrmals täglich durchgeführt wird, kann mittels herkömmlicher Methoden erfolgen, d.h. mit herkömmlichem pH-Papier oder einem pH-Meter. Eine bevorzugte Messmethode unter Verwendung von Hygieneartikeln mit pH-Indikatoren ist in DE 20 2007 015 088.1 und DE 20 2007 016 404.1 beschrieben.

Die nachfolgenden Beispiele veranschaulichen den Gegenstand der Erfindung.

### Beispiel 1

### Herstellung von Zubereitungen

Eine erfindungsgemäße Zubereitung enthält im Verhältnis

| | |
|---|---|
| Natrium, | 135-145 mmol |
| Kalium, | 3,5-5,0 mmol |
| Calcium, | 2,05-2,65 mmol |
| Magnesium, | 0,65 - 1,10 mmol |

Diese Elektrolyte liegen als Hydrogencarbonate oder/und Citrate vor. Bevorzugt liegt Natrium als Natriumhydrogencarbonat aber auch als Natriumcitrat vor. Kalium, Calcium und Magnesium liegen bevorzugt als Citrat vor. Besonders bevorzugt ist eine Zusammensetzung, die Natriumhydrogencarbonat, Kalium-, Calcium- und Magnesiumcitrat enthält.

Die Zubereitung wird oral, z.B. in Tablettenform, täglich gegebenenfalls mehrfach verabreicht. Die Dosierung wird aufgrund von Messungen des pH im Urin ermittelt. Die Dosierung wird vorzugsweise so eingestellt, dass der pH permanent einen Wert ≥ 7,0, vorzugsweise 7,0-7,5, aufweist.

### Beispiel 2

### Bestimmung von Kreatininwerten

Es wurde die Hemmung des Fortschreitens von chronischem Nierenversagen vermittels langfristiger oraler Verabreichung von Bicarbonat zur Einstellung eines alkalischen pH-Werts im Urin (Bicarbonat Alkalische Polyuria/BAP) untersucht. Hierzu wurden Kreatininwerte in mehreren Patienten bestimmt.

### Die Ergebnisse sind in der folgenden Tabelle 1 gezeigt.

| **Pat. Nr.** | **Geschl.** | **BAP Start** | **Start-Alter in Jahren** | **RR mmHg** | **Kreatinin Start mg/dl** | **Aug. 2000 Alter** | **RR mmHg** | **Aug. 2000 Kreatinin mg/dl** | **Behandlungdauer** |
|---|---|---|---|---|---|---|---|---|---|
| 1 | m | Dez. 1973 | 29 J. | 150/90 | 1,4 | 56 J. | 120/75 | 1,2 | 27J |
| 2 | m | Mai 1975 | 56 J. | 200/90 | 1,0 | 81 J. | 140/80 | 1,9 | 25 J. |
| 3 | m | Jan. 1977 | 54 J. | 180/90 | 1,4 | 77 J. | 140/90 | 1,3 | 23 J. |
| 4 | m | Okt. 1981 | 53 J. | 200/140 | 1,6 | 72 J. | 120/80 | 2,1 | 19 J. |
| 5 | m | Apr. 1987 | 50 J. | 165/85 | 3,0 | 63 J. | 130/75 | 1,8 | 13 J. |
| 6 | f | Apr. 1988 | 48 J. | 170/110 | 0,9 | 61 J. | 110/80 | 1,3 | 12 J. |
| 7 | f | Nov. 1991 | 31 J. | 130/80 | 0,9 | 39 J. | 100/65 | 0,6 | 9 J |
| 8 | m | Feb. 1994 | 64 J. | 150/90 | 3,7 | 71 J. | 130/80 | 2,5 | 6 J. |
| 9 | f | Juli 1994 | 58 J. | 150/80 | 1,6 | 64 J. | 140/85 | 1,2 | 6 J. |
| 10 | m | Nov. 1996 | 50 J. | 120/80 | 1,1 | 54 J. | 140/90 | 0,7 | 4 J. |
| 11 | f | Juli 1997 | 46 J. | 140/05 | 1,4 | 49 J. | 135/80 | 1,6 | 3 J. |
| 12 | f | Okt. 1997 | 32 J. | 160/110 | 0,7 | 35 J. | 115/70 | 0,7 | 3 J. |
| 13 | m | Dez. 1997 | 78 J. | 150/90 | 2,8 | 81 J. | 130/70 | 3,0 | 3 J. |

Es ist zu erkennen, dass bei den untersuchten Patienten der Kreatininwert vom Beginn der Therapie bis ins Jahr 2000, d.h. über einen Zeitraum von 3-27 Jahren, nicht signifikant zugenommen hat. Insbesondere bei Patienten mit höherem Kreatininwert (≥ 3 mg/dl) zu Beginn der Therapie wird eine Abnahme beobachtet.

### Beispiel 3

### Bestimmung der Knochendichte

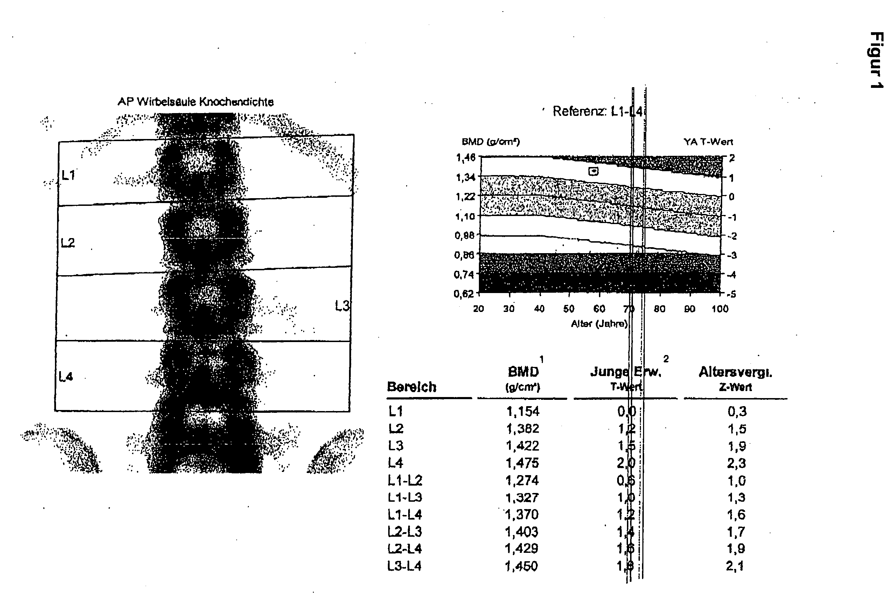

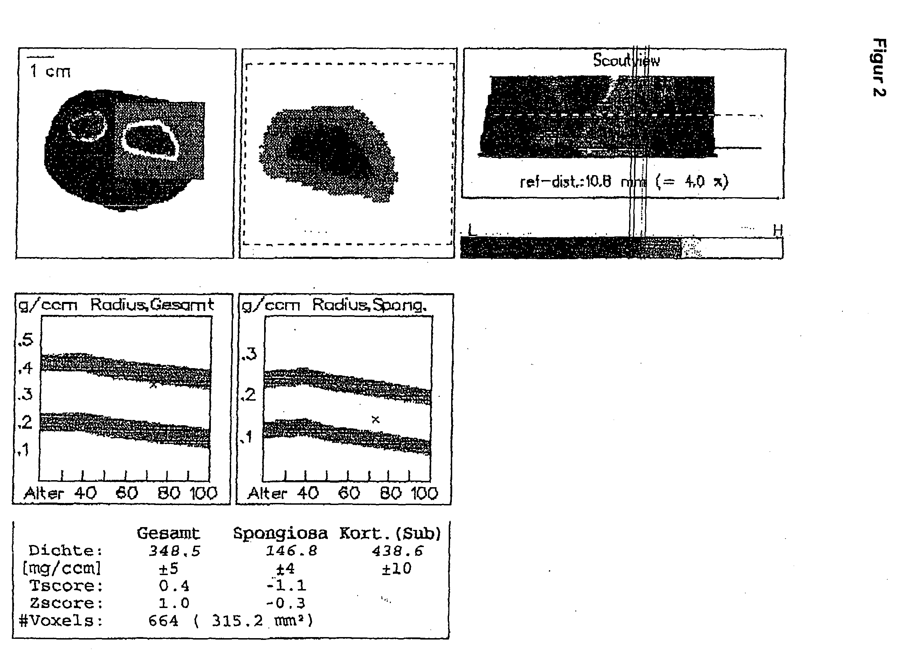

Befunde zur Knochendichte sind für die Patienten 1 (Figur 1) und 4 (Figur 2) aus Beispiel 2 gezeigt. Es ist ersichtlich, dass beide Patienten nach mehrjähriger Dauer der BAP-Behandlung (27 bzw. 19 Jahre) keine pathologischen Veränderungen der Knochendichte aufweisen.

Für eine weitere Patientin (Patientin 14) wurde nach 24-jähriger Dauer der BAP-Behandlung im Jahr 2007 folgender Befund zur Osteodensitometrie (durch quantitative Mehrzahlencomputertomographie) erhoben.

### Befund:

Die trabekuläre Knochenmineralsalzdichte beträgt in LWK 2 137,0 mg Calciumhydroxylapatit/ml, in LWK 3 140,0 mg/ml und in LWK 4 145,0 mg/ml.

Aus diesen Einzelwerten errechnet sich ein mittlerer Knochenmineralsaldichtewert von 141,0 mg Calciumhydroxylapatit/ml +/- 3. Alters- und geschlechtsspezifischer Referenzwert 93,0 mg/ml. (Gemäß WHO Definition liegt > 120 mg/ml eine Osteopenie, < 80 mg/ml eine Osteoporose vor). Der errechnte T-score analog liegt bei 0,65, der Z-score analog bei + 1,7.

### Makromorphologisch jugendliches Achsskelett ohne Frakturdemarkierung.

### Beurteilung:

Hochnormaler Knochenmineralsalzgehalt. Keine Osteopenie oder Osteoporose.

## Patentansprüche

1. Verwendung einer Zubereitung, enthaltend als Wirkstoff mindestens ein Hydrogencarbonatsalz oder/und mindestens eine Verbindung, die im Körper zu einem Hydrogencarbonatsalz metabolisiert wird, zur Herstellung eines Mittels zur Prävention oder Behandlung von Zivilisationskrankheiten wie etwa Herz-Kreislauf-Erkrankungen, Krebserkrankungen, Bluthochdruck, Diabetes, Osteoporose, chronischem Nierenversagen und zur Abwendung eines im Rahmen anderer Erkrankungen möglichen akuten Nierenversagens,
**dadurch gekennzeichnet,**
**dass** die Zubereitung zur Verabreichung in einer Dosierung vorgesehen ist, durch die der pH des Urins auf einen Wert ≥ pH 7,0 eingestellt wird.

2. Verwendung nach Anspruch 1,
**dadurch gekennzeichnet,**
**dass** die Zubereitung zur Verabreichung in einer Dosierung vorgesehen ist, den pH des Urins permanent auf einen Bereich von pH 7,0 bis pH 7,5 einzustellen.

3. Verwendung nach Anspruch 1 oder 2,
**dadurch gekennzeichnet,**
**dass** die Zubereitung zur oralen Verabreichung vorgesehen ist.

4. Verwendung nach einem der Ansprüche 1-3,
**dadurch gekennzeichnet,**
**dass** die Zubereitung als Wirkstoff Natriumhydrogencarbonat, Kaliumhydrogencarbonat, Calciumhydrogencarbonat, Calciumcitrat, Natriumcitrat, Kaliumcitrat oder/und Magnesiumcitrat enthält.

5. Verwendung nach einem der Ansprüche 1 bis 4,
**dadurch gekennzeichnet,**
**dass** die Zubereitung als Wirkstoff (i) Natriumhydrogencarbonat oder/und Natriumcitrat, (ii) Kaliumcitrat, (iii) Calciumcitrat und (iv) Magnesiumcitrat enthält.

6. Verwendung nach einem der Ansprüche 1-5,
**dadurch gekennzeichnet,**
**dass** die Zubereitung kationische Elektrolyte im Wesentlichen entsprechend dem Verhältnis ihres Vorkommens in Humanserum enthält.

7. Verwendung nach Anspruch 6,
**dadurch gekennzeichnet,**
**dass** die kationischen Elektrolyte aus Na, K, Ca und Mg ausgewählt werden.

8. Verwendung nach Anspruch 7,
**dadurch gekennzeichnet,**
**dass** das Molverhältnis Mg:Ca:K:Na im Bereich von 1:1,5-5:3-10:100-250 liegt.

9. Verwendung nach einem der Ansprüche 1 bis 8,
**dadurch gekennzeichnet,**
**dass** die Zubereitung ein Getränk, ein Pulver, ein Bonbon, eine Tablette, eine Kapsel oder ein Riegel ist.

10. Verfahren zur Prävention oder Behandlung von Zivilisationskrankheiten wie etwa Herz-Kreislauf-Erkrankungen, Krebserkrankungen, Bluthochdruck, Diabetes, Osteoporose, chronischem Nierenversagen und zur Abwendung eines im Rahmen anderer Erkrankungen möglichen akuten Nierenversagens, das die folgenden Schritte umfasst:
(a) Verabreichung einer wie in einem der Ansprüche 1-9 definierten Zubereitung,
(b) Messung des pH-Wertes des Urins,
(c) erneute Verabreichung der Zubereitung, falls der gemessene pH des Urins < pH 7,0 beträgt, und Wiederholung der Verabreichung und Messung des pH des Urins, bis der pH des Urins ≥ pH 7,0 ist.

11. Verfahren nach Anspruch 10,
**dadurch gekennzeichnet,**
**dass** die Verfahrensschritte (a) bis (c) wiederholt werden, bis der pH des Urins permanent in einem Bereich von pH 7,0 bis pH 7,5 liegt.

12. Verfahren nach einem der Ansprüche 10-11,
**dadurch gekennzeichnet,**
**dass** die Verabreichung kontinuierlich, insbesondere täglich, erfolgt.

13. Verfahren nach einem der Ansprüche 10-12,
**dadurch gekennzeichnet,**
**dass** eine langfristige Verabreichung erfolgt.
